# EUROPEAN PATENT APPLICATION

(11) **EP 4 531 049 A1**
(43) Date of publication of application: **02.04.2025**
(21) Application number: 23200739.3
(22) Date of filing: 29.09.2023
(51) Int. Cl.: G16H 10/60, G06N 20/00, G16H 20/70, G16H 40/63, G16H 50/20, G16H 50/70, G06N 3/08

(54) **METHOD FOR SELECTING PATIENT EXPERIENCE CONTENT AND PATIENT EXPERIENCE CONTENT SELECTION ENGINE**

(71) Applicant: Koninklijke Philips N.V., 5656 AG Eindhoven (NL)
(72) Inventor: SCHADEWALDT, Nicole, Eindhoven (NL); NAUTS, Sanne, Eindhoven (NL); VAN DER VLEUTEN-CHRAIBI, Sanae, 5656AG Eindhoven (NL); SAINI, Privender Kaur, Eindhoven (NL); HEUVELINK, Annerieke, Eindhoven (NL)
(74) Representative: Philips Intellectual Property & Standards

(57) **Abstract**

The invention relates to a method for selecting patient experience content for a medical procedure. According to the method, a library (6) comprising a plurality of patient experience content items is provided (S 1) and low level content properties for each of the patient experience content items are obtained (S2). Then, high level content effects are derived (S3) from the low level content properties for each of the patient experience content items and the patient experience content items are prioritized (S4) based on their high level content effects and a target effect. Finally, a selection of patient experience content items is generated (S5) based on the prioritized (S4) patient experience content items. The invention further relates to a patient experience content selection engine (5) and a medical apparatus (1) comprising said patient experience content selection engine (5).

## Description

### FIELD OF THE INVENTION

The invention relates to medical procedures and, in particular, to a method for selecting patient experience content for a medical procedure. The invention further relates to a patient experience content selection engine and to a medical apparatus comprising said patient experience content selection engine.

### BACKGROUND OF THE INVENTION

For patients, undergoing a medical procedure (e.g., a radiological procedure such as an MRI-scan or CT-scan) can be a stressful, anxiety-provoking experience. Moreover, many radiological exams and other medical procedures require that a patient complies with certain instructions or keeps very still during the exam. As an example, if patients need to undergo an MRI-scan, they need to keep very still during the scan as even minor movement can negatively affect the diagnostic quality of the scan, which may result in the need to repeat the scan.

To improve patient's experience, engagement and compliance with instructions (e.g., the instruction to keep their body still during a scan or exam, or follow breathing instructions), several systems exist that can be used to present patient experience content (such as movies, pictures, sounds, music, audiobooks and light effects) to patients. This content can be presented to patients using different types of equipment, such as screens, projections, sound systems, light fixtures, etc. Engaging with patient experience content (e.g., watching a movie or listening to music) during an exam may help patients feel less anxious and make it easier to keep still, which can potentially improve the experience of patients and staff, as well as the hospital's workflow and the quality of scans or procedures.

Patient experience content is designed to improve the experiences of patients and staff, as well as, e.g., the flow of magnetic resonance imaging (MRI) examinations and other medical imaging procedures and potentially other diagnostic or therapeutic procedures. Patient experience content typically consists of visuals (e.g., a movie or slideshow with pictures), sound (stand-alone or integrated with the visuals), and light settings, but it can also contain other elements such as olfactory cues (smells) or tactile cues. Patient experience content has shown to have multiple positive effects, including calming down the patient, reducing anxiety and increasing comfort. It can also increase sense of control when the patient can choose the patient experience content to accompany them during the procedure.

However, for the patient experience content to work, two properties must be met. First, the patient experience content, e.g., a video, must be suitable to produce the desired effects like calming, engaging, or distracting, and not trigger unwanted effects like head movements or anxiety/startle reactions, which may trigger motion, or falling asleep which limits the patients to follow e.g., breathing instructions. Then, the patient must like and/or appreciate the content, to prevent disengagement, agitation or annoyance.

In current clinical practice, the staff can present the patient with a manageable list of content options with desired properties to choose from. To save time and prevent choice overload, this list should be short, e.g., comprising five to eight content items. If the list is too large, or the clinical staff cannot decide on the selection to offer to patients, the clinical staff often ends up choosing the content for the patient. Also, if the patient is presented with too much suitable content, they are likely to be less satisfied by their choice as they miss out on other potentially good options. Thus, providing a limited selection is preferable also for patient satisfaction. Typically, this is content that has proved valuable from the clinician's perspective and experience to be acceptable to most patients.

An alternative approach that is employed by the staff is to use the patient experience content delivery equipment (e.g., screen; projector; lights) to display patient preferred content from third party sources. This may be done because, e.g., providing a suitable selection of content to a patient is too laborious given the amount of content items available, or because it is felt that the library of content provided by the patient experience system is too limited to fulfill the patient's preference (e.g. wanting to see the latest episode of their favorite show on e.g., a streaming service or online content platform). To still provide patients with a sense of control and personalization, staff often lets patients tell them what they want to watch during the medical procedure. Patients may select popular content from third party libraries, e.g., from streaming services. However, these content libraries contain content that may not be suitable to be used during the medical image procedure like an MRI-scan: for example, it can contain content that increases anxiety within this specific context, such as scary imagery, or imagery of patients in hospitals, or content that increases the likelihood of movement, or content that is disorienting in the context of the medical procedure. The situation in the hospital, e.g., within an MR scanner is very different to sitting at home on the sofa, so the patient selected content might be less suitable for this setting. Also, within an MR scanner audio components of the content are only partially audible, as the loud MR noise overpowers the content-related audio. Thus, content where audio is essential, e.g., dialogues, is less suited. Moreover, content that is fast-paced and has certain features can negatively affect people's goal-directed behavior and ability to focus. Examples of features that can have these effects are fast-paced movement of character, frequent camera movement, frequent editorial transitions between shots (e.g., zooms, pans, cuts, fades, wipes), flicker, and rapid changes in low-level visual features such as changes in luminance or color. For music, features such as the number of Beats Per Minute (BPM) can affect people's emotional response (e.g., feeling calm) as well as their physiology (e.g., heart rate, blood pressure). These kinds of low-level content features can be measured using various (software) tools, or can be rated by experts.

In essence, currently there is no good way of giving patients a choice of appropriate and appreciated content, i.e., content that facilitates cooperation and relaxation and content that is liked by the patient.

Since the patient experience and the quality of the medical procedures, e.g., the MRI exam quality, can be improved significantly by showing suitable content to patients to reduce anxiety and motion and have other positive effects on patient behavior and feelings, several approaches to select such suitable content have been made. As one approach, a small library of suitable content may be created. However, this may not hold suitable content for every patient, since the selection is too small; it may also not be suitable for every medical procedure, since procedures widely vary in length, and the content may not be of the same length as the procedure. And even though such a library may contain a relatively small amount of content, e.g., only 45 movies, this may already be too large for a thorough browsing and selection by each patient. As an alternative approach, the right content may be selected from a library of suitable content. This becomes less efficient the larger the library is, however, the smaller the library is, the more likely it is that it is impossible to find a match for every patient in every medical procedure situation. As yet an alternative approach, the right content may be selected from a large library of unclassified content, like large streaming providers. However, given a huge library of content, it is impossible to select all suitable content manually.

Further, it is impossible for the patient to choose a best video from a library for two reasons: first, the sheer amount of videos/audio content, and second, the lack of experience of effects within this particular situation (in the context of a scan or other medical procedure). The patient is likely able to select many videos or pieces of music from a streaming provider, however, it is much less likely, that the patient will take the effect properties into account to choose from those, what would be most helpful.

Moreover, when considering tactile or olfactory content (standalone or as add-on to audiovisual content) it is also not likely for a patient (or staff) to select this on the spot as understanding the content may be hard and inferring the effect it may have can be even more challenging.

### SUMMARY OF THE INVENTION

It is therefore an object of the present invention to provide a method that solves the above-mentioned problem, in particular to provide a method for selecting patient experience content for a medical procedure. It is a further object of the present invention to provide a corresponding patient experience content selection engine and a medical apparatus comprising said engine.

The object of the present invention is solved by the subject matter of the independent claims, wherein further embodiments are incorporated in the dependent claims.

In an aspect of the present invention, a method for selecting patient experience content for a medical procedure is provided. In this context, patient experience content refers to any content that is presented to the patient during the medical procedure.

According to the method, a library comprising a plurality of patient experience content items is provided. Said library may comprise a digital library, which may be either local and/or remote. Alternatively, or additionally, the library may comprise an analog library.

For each of the patient experience content items, low level content properties are obtained, such as spatial orientation, velocity, color-change speed, viewpoint orientation, zooms, pans, spatial contrast, color contrast, color tone, flicker, pace, of which more examples will follow below. The low level content properties may also be referred to as low level features, as sensorial qualities and/or contextual qualities and may include properties that can be obtained for an individual patient experience content item without knowledge of the later application, i.e., without knowing the effects that these features may have. For each of the low level content properties, a scale may be chosen, e.g., a calibrated scale between zero and one.

From said low level content properties, high level content effects are derived for each of the patient experience content items. Then, the patient experience content items are prioritized based on their high level content effects and a target effect. The prioritization is based on how likely the patient experience content items are to achieve the target effect. Said target effect may also be called desired effect and may depend, e.g., on the particular medical procedure. The target effect may be selected by the staff, may be based on information regarding the medical procedure, e.g., on scan information, or may be pre-determined, in particular for specific medical procedures. Examples for target effects are relaxation, leading to a less aroused state, leading to a state of interest, curiosity and/or engagement. The target effect may be chosen such that the patient behaves better during the medical procedure, e.g., lies still, follows instructions, controls panic, breaths in a desired pattern, and/or has a positive experience that enables her to receive a follow-up medical procedure, e.g., a follow-up medical scan, with the same optimal behavior. Of course, the target effect may comprise a plurality of effects, e.g., the target effect may be to both reduce anxiety and to slow down breathing.

Based on the prioritized patient experience content items, a selection of patient experience content items is generated. Said selection may comprise only a small number of suggested patient experience content items, e.g., only three to five items. Hence, a huge amount of potential patient experience content items is reduced to a shortlist of favorites that take the target effect into account. The method reduces the list of patient experience content suggestions, such that it is much easier for the patient to select from the list of patient experience content items, while relevant options are ensured to maximize the sense of control. Alternatively, the selection may be done by a third person for the patient, e.g., a caretaker of the patient (parents, spouse), or in some cases the staff, for which third person the selection would also be easier given the limited choice. In particular, the selected patient experience content items have the desired effect, i.e., the target effect, on the patients. Finally, since the library may be huge, it is unlikely that too little suitable content may be found for specific patient groups, e.g., based on patient properties like age or gender, and/or on properties of the medical procedure, e.g., a scan duration or a type of scan, and/or preferences indicated by the patient, e.g., topics such as "animals", "adventure", "nature", "travel", "art", and/or exclusion criteria defined by the patient, e.g., certain animals or conditions they are afraid of. The patient's input may be enabled by choosing from a list, both preferences and avoidances, prior to the exam, e.g., in the waiting room or at home. However, patient input is just one possible embodiment, the system may also work without patient input.
Further, previously chosen patient experience content may be taken into account for the prioritization. This means if e.g., the patient had a prior CT exam, the content chosen for that exam might be valued highly on the priority list, or if there are options to choose experience content in preparatory settings, e.g., in the waiting room or while receiving instructions for the scan, e.g., breath-hold instructions, this prior choice might be taken into account for prioritization as well.

According to an embodiment, the medical procedure is a medical imaging procedure. Examples for said medical imaging procedures are magnetic resonance imaging (MRI) procedures, computed tomography (CT) procedures, X-ray procedures (incl. fluoroscopy), Ultrasound, or imaging taking place as part of an interventional procedure, e.g., image guided therapy (IGT). Alternatively, or additionally, the medical procedure is a medical therapy procedure, such as an IGT procedure, or a radiotherapy procedure. In said medical procedures, patient experience content is very useful for improving the patient experience, the medical workflow, and/or the quality of the medical procedure, e.g., the quality of the scans or the performance of the therapy. Of course, the patient experience content may also be used for other medical procedures, such as reducing patient anxiety for patients with a delirium, reducing fear in an emergency room, or reducing boredom or apprehension in a waiting room.

According to an embodiment, the patient experience content comprises visual content, auditory content, olfactory content, and/or tactile content. Visual content may be, in particular, videos. In this case, the video library may include videos that are specifically designed to help with a particular medical procedure, e.g., with an MRI exam experience. Alternatively, or additionally, the video library may include videos with soothing content for more general applications, e.g., nature videos. Alternatively, or additionally, the video library may include movies, DVDs, and/or online content, e.g., from streaming providers. Likewise, the auditory content may be obtained from a variety of sources, e.g., specifically designed music, music from CDs, music from streaming providers, audiobooks, radio plays, nature sounds (wind, waves, rustling leaves, ...) and/or the soundtrack of the videos. In particular, videos may be chosen as patient experience content items with the corresponding soundtrack, but it is also possible to choose only the visual content of the videos, and/or choose the visual content of the videos and provide different auditory content, e.g., replace voices by soothing music. Olfactory content may be chosen from a selection of possible odors, such as pine, cinnamon, coconut, rose, and sea salt, and may be matched to the visual content, e.g., tropical fruit odors with a tropical video theme. Likewise, tactile content may be chosen from a set of possible tactile experiences, such as furry, sleek, felt, leather, and wood, and may be matched to the visual or audio content. Having one or several of the above-mentioned patient experience contents will help to achieve the target effect.

According to an embodiment, the low-level content properties comprise color, spatial orientation, velocity, color-change speed, viewpoint orientation, zooms, pans, spatial contrast, color contrast, color tone, flicker, pace, intensity of movement, fades, edge density, average shot length, discontinuous edits, cuts, faces contained, and/or presence of predefined content for visual content. In this context, the velocity may be an average velocity and/or a peak velocity. The flicker may be defined as a change in luminance over time, and, in particular, an amount of flicker may be determined. The pace of the visual content may be defined as a number of cuts in the video. The average shot length is understood to be an average length between cuts. The term "discontinuous edits" refers to the number of scene changes, e.g., per amount of time. The predefined content may be, e.g., animals, wherein, e.g., bugs, spiders or snakes are known to trigger phobia in patients, whereas cats trigger soothing feelings and enjoyment for most patients. Other predefined content may be heights or confined spaces such as caves that may also trigger phobia in patients. For auditory content, the low level content properties may comprise language and/or audio changes, e.g., changes in tone or volume; it may also contain auditory or psychoacoustic parameters such as loudness, sharpness, roughness, rhythm, tempo, e.g., beats per minute (BPM). For olfactory content, the low level content properties may comprise an intensity of odor, i.e., a strength of fragrance, a type of odor, e.g., floral, musk, and/or peppermint, odor familiarity, odor valence, and/or pre-existing odor associations, such as citron for clean. For tactile content, the low level content properties may comprise roughness, in particular macro roughness and/or fine roughness, temperature, i.e., warmness and/or coldness, including heat capacity and heat conductance, hardness, and/or friction, e.g., moistness, dryness, stickiness and/or slipperiness. Hence, a large amount of low level content properties can be obtained, making the determination of the high level content effects more reliable.

According to an embodiment, the step of obtaining low level content properties comprises computing the low level content properties. Said computation of the low level content properties may be performed by an explicit computation engine. For many of the above-mentioned low level content properties, methods for explicit computation exist and/or can be easily implemented. Alternatively, or additionally, the low level content properties may be computed using implicit knowledge, e.g., as captured by a trained neural network. In particular, said trained neural network has been trained with a plurality of patient experience content items with a labelled ground truth, wherein the labels differentiate, e.g., between several classes of videos.

Alternatively, or additionally, the low level content properties can be obtained by manually annotating the patient experience content items, which may be preferable, e.g., for olfactory content. Yet alternatively, or additionally, the low level content properties may be derived from information provided by a third party, for example the well-known age-appropriateness suggestions for movie content available in many countries (PG-recommendations in the USA; FSK-rating in Germany), along with any existing tagging of content (e.g., sensitivity labels such as including violence, sexually explicit content, fear-inducing content, or genres like family, science-fiction, horror, etc.).

According to an embodiment, the high level content effects comprise physical comfort, calming effects, re-assuring effects, feeling safe effects, sensory indulgence effects, engaging effects, distracting effects, and/or fear-inducing effects. As an example, calming effects may be required for an MRI exam or radiotherapy procedure, where the patient shall relax and stay still. Said calming effects correspond to low level properties with slow pacing, few cuts, little flicker, absence of sudden and/or loud noises, and/or little imagery at edges of the screen. As another example, high level content effects that are to be avoided are fear-inducing effects such as violent content, situations and/or animals that can cause fear in certain patients, such as heights and/or spiders, and/or situation-specific things that can provoke fear, such as imagery of hospitals, illness, or confined spaces, e.g., caves and elevators. From these high level content effects, the target effect(s) can be chosen, such that an optimal prioritization of the patient experience content items can be achieved.

According to an embodiment, deriving the high level content effects is performed using a trained neural network. Said neural network has been trained using a plurality of training content items that have been labelled with high level content effects. In this context, the training content items may be a selection of the patient experience content items. Said labelling may have been performed by experienced staff who can predict the high level content effects from the low level content properties. Alternatively, high level content effects may be measured when the training content items are presented to test persons. Said measuring of the high level content effects may be performed by measuring, e.g., heart rate, skin temperature and/or eye movement of the test persons. Once the neural network has been trained, it can be easily applied to the plurality of patient experience content items, yielding the high level content effects for all of the plurality of patient experience content items. As an alternative, or additionally, to the trained neural network, another artificial intelligence and/or another algorithm may be used. In particular, methods using implicit knowledge on the relationship between low level content properties and high level content effects may be used. As another alternative, systems that use explicit knowledge, such as expert systems or ontology, may be used to derive the high level content effects from the low level content properties. As yet another alternative, models that use both implicit and explicit knowledge, such as Bayesian networks, may be used to derive the high level content effects from the low level content properties.

According to an embodiment, deriving the high level content effects is further based on patient information and/or on situational information. Said patient information may comprise age, cultural background, interests, gender, and/or prior medical exams. The situational information is, e.g., information on the situation of the medical procedure. As an example, a situation information on an MRI exam is that it is accompanied with loud noise. Given said situation information, the helpful patient experience content has to be independent of supporting auditory content, specifically no voice or dialogue, and should induce calmness to counteract the noise. As another example, the situational information may comprise information that the patient is lying down. In this case, visual content should not be disorienting, e.g., characterized by low level properties specifying that the content has a horizon or a similar form of orientation, i.e., grounding features. If the high level content effects are derived using a trained neural network or another artificial intelligence, the patient information and/or situation information is also to be considered in the training. For example, calming effects may be different for different types of exams, feeling safe may depend on the age of the patient, and activating effects may depend on the type and/or length of an exam. In addition to the patient information and/or situation information, other types of information may be used to derive the high level content effects, e.g., databases comprising an age appropriateness of videos and/or music. With the additional information taken into account, the derivation of the high level content effects, and consequently the effects on the patient and the medical procedure are further improved.

According to an embodiment, prioritizing the patient experience content items is further based on patient preference. Said patient preference may be provided by the patient, e.g., by indicating what kind of scenes or videos they like or do not like. For example, the patient can be offered a checklist of properties and label those, which he/she likes or which make him/her feel relaxed and calm, e.g. "nature", "challenge", "vacation", "animals", "culture", "colorful", "water". In this case, the number of likes may be limited, or the patient may be asked to prioritize the categories. As another example, the patients may bring in their user preference profiles, e.g., through their personal device or by providing their social media account information. If none of the above information is provided, then general patient information, such as age and/or gender may be used to prioritize the patient experience content items based on what patient with a similar profile liked. By taking the patient preferences into account in the selection of the patient experience content items, it is more likely that patient experience content items that the patient likes will be selected, which will then further improve the positive effect of the patient experience content items on the patient and on the medical procedure.

According to an embodiment, prioritizing the patient experience content items is further based on medical procedure information. Said medical procedure information may comprise a length of the medical procedure, a requirement to lie still, an expected patient cooperation, an expected noise level, and/or the use of contrast. As an example, the length of the planned procedure could be used to filter content in the prioritizing step to propose only content of matching length. Moreover, if a patient has a preference for audiovisual content that consists of multiple parts or episodes, or that is longer than the medical scan or medical procedure, the most optimal episode or part of that content can be selected. In particular, a movie may be shown that does not start at the beginning and/or that skips parts of the movie that have undesirable high level content effects. In the above context, the expected patient cooperation or response may be, e.g., with respect to breathing rate/amplitude, heart rate, and/or to moving. Taking the medical procedure information into account, an even better selection of patient experience content items may be achieved.

According to an embodiment, generating the selection of patient experience content items comprises using a similarity measure of the patient experience content items, such that the selection of patient experience content items comprises types of patient experience content items having little similarity. In other words, the similarity measure can be employed to suggest different types of patient experience content items. Hence, the patient has a wider range to select the patient experience content items and it is therefore more likely that patient experience content items that the patient likes are included in the selection. As an extreme example, if a 5 hour video of beach scenes is considered the most suitable content for a 20 min procedure, any 20 min section of this video would be suitable, and the tops 5 content items provided to the patient might be all from this video. This is not useful and the similarity measure should avoid that.

According to an embodiment, the steps of computing low level content properties, deriving high level content effects, and/or prioritizing the patient experience content items are performed using machine learning, and the machine learning is trained using medical procedure workflow data. In particular, the machine learning approach may be an artificial intelligence such as a neural network. The medical workflow data may comprise data such as a number of rescans that were performed for specific content, as well as a number of scan aborts, or a total scan time. This medical workflow data can then be used as a measure of what content helps the patients to have a successful medical procedure. This is then used as training input for the machine learning system. With said procedure, the real effect on patients during the medical procedure is evaluated and used to determine selections of patient experience content items, which then have an even better positive effect on the patient and/or the medical procedure.

According to an embodiment, the method further comprises the steps of presenting the selection of patient experience content items to a patient, receiving input on the choice from the patient; and providing the chosen patient experience content item to the patient during the medical procedure. Hence, the selection of the patient experience content items is actually used to provide one patient experience content item to the patient during the medical procedure, leading to positive effects on the patient and/or on the result of the medical procedure.

In another aspect of the present invention, a patient experience content selection engine is provided. Said patient experience content selection engine may comprise a computing unit, comprising at least one processor. The patient experience content selection engine may further comprise a library comprising a plurality of patient experience content items and/or may be connected, e.g., via a wired or wireless connection, to a library comprising a plurality of patient experience content items. The patient experience content engine is configured to perform the method according to the above description. Hence, the patient experience content engine reduces a huge amount of potential patient experience content items to a shortlist of favorites that take the target effect into account. The patient experience content engine reduces the list of patient experience content suggestions, such that it is much easier for the patient to select from the list of patient experience content items, while relevant options are ensured to maximize the sense of control. In particular, the selected patient experience content items have the desired effect, i.e., the target effect, on the patients. Finally, since the library may be huge, it is unlikely that too little suitable content may be found for specific patient groups, e.g., based on patient properties like age or gender, and/or on properties of the medical procedure, e.g., a scan duration or a type of scan.

In yet another aspect of the present invention, a medical apparatus comprising a patient experience content selection engine according to the above description is provided. Said medical apparatus may be a magnetic resonance imaging, MRI, apparatus, a computed tomography, CT, apparatus, an image guided therapy, IGT, imaging apparatus, an IGT therapy apparatus, or a radiotherapy apparatus. Advantages, embodiments, and further details of the medical apparatus correspond to those given in the above description of the method for selecting patient experience content for a medical procedure and the patient experience content selection engine.

It shall be understood that a preferred embodiment of the invention can also be any combination of the dependent claims with the respective independent claim. Further, it has to be noted that embodiments of the invention are described with reference to different subject matters. In particular, some embodiments are described with reference to device type claims whereas other embodiments are described with reference to method type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematic perspective view of an embodiment of a medical apparatus; and
Fig. 2 shows a flowchart of an embodiment of a method for selecting patient experience content; and
Fig. 3 shows a flowchart of another embodiment of a method for selecting patient experience content.

In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

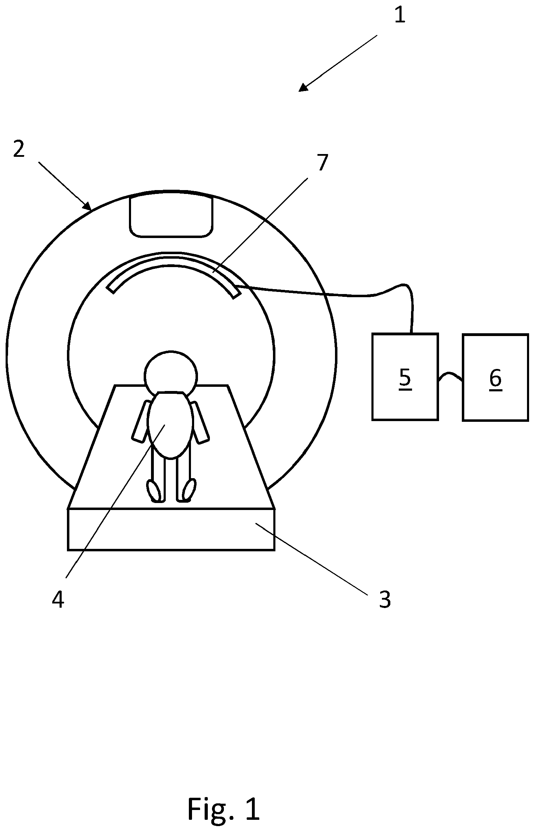

Fig. 1 shows a schematic perspective view of an embodiment of a medical apparatus 1. The medical apparatus 1 comprises a medical device 2, which is shown as a magnetic resonance imaging (MRI) device. However, also other medical devices 2 may be used, such as computed tomography (CT) devices, image guided therapy (IGT) imaging devices, IGT therapy devices or radiotherapy devices. In the present embodiment, the medical apparatus 1 further comprises a patient table 3, on which a patient 4 is shown.

The medical apparatus 1 also comprises a patient experience content selection engine 5. Said patient experience content selection engine 5 is configured to select patient experience content for a medical procedure, in the present embodiment for an MRI scan. The patient content selection engine 5 is connected to a library 6 comprising a plurality of patient experience content items. In an alternative embodiment, which is not shown here, the patient experience content selection engine 5 may comprise the library 6.

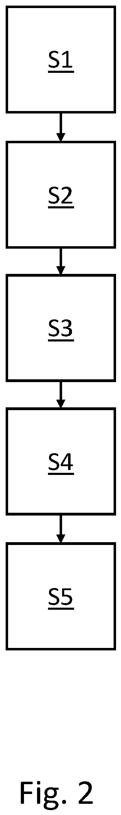

The patient experience content selection engine 5 selects a patient experience content item according to the method described below in Fig. 2. Once the patient experience content item has been selected, it will be provided to the patient 4 during the medical procedure. In the present embodiment, this is done by displaying visual content on a screen 7 of the medical apparatus 1. Other content may be provided to the patient 4, too, e.g., auditory content, olfactory content, and/or tactile content.

The method according to which the patient experience content selection engine 5 is operated is shown in Fig. 2.

According to the method, the library 6 comprising the plurality of patient experience content items is provided, denoted as S1 in Fig. 2. Then, low level content properties are obtained S2 for each of the patient experience content items. Said low level content properties may comprise, depending on the type of patient experience content, color, spatial orientation, velocity, color-change speed, viewpoint orientation, zooms, pans, spatial contrast, color contrast, color tone, flicker, pace, intensity of movement, fades, edge density, average shot length, discontinuous edits, cuts, faces contained, presence of predefined content, language, audio changes, intensity of odor, type of odor, roughness, temperature, hardness, and/or friction. The low level content properties may be obtained S2 by computing the low level content properties, in particular by an explicit computation engine and/or by a trained neural network.

Then, high level content effects are derived S3 from the low level content properties for each of the patient experience content items. Said high level content effects may comprise physical comfort, calming effects, re-assuring effects, feeling safe effects, sensory indulgence effects, engaging effects, distracting effects, and/or fear-inducing effects. In particular, the high level content effects may be derived S3 using a trained neural network, wherein the neural network has been trained using a plurality of training content items that have been labelled with high level content effects. In order to further improve the high level content effects, deriving S3 the high level content effects may be further based on patient information and/or on situation information.

Once the high level content effects have been derived S3, the patient experience content items are prioritized S4 based on their high level content effects and on a target effect. Said target effect may be selected by the staff, may be based on information regarding the medical procedure, e.g., on scan information, or may be pre-determined, in particular for specific medical procedures. Examples for target effects are relaxation, leading to a less aroused state, leading to a state of interest, curiosity and/or engagement. The target effect may be chosen such that the patient behaves better during the medical procedure, e.g., lies still, follows instructions, controls panic, and/or is able to receive a follow-up medical procedure, e.g., a follow-up medical scan, with the same optimal behavior. Of course, the target effect may comprise a plurality of effects, e.g., the target effect may be to both reduce anxiety and to slow down breathing. Prioritizing S4 the patient experience content items may be further based on patient preference, in order to obtain patient experience content items that the patient likes. Further, prioritizing S4 the patient experience content items may be based on medical procedure information, in particular a length of the medical procedure, requirement to lie still, expected patient cooperation, expected noise level, and/or use of contrast.

Based on the prioritized S4 patient experience content items, a selection of patient experience content items is generated S5. Said selection may comprise only a small number of suggested patient experience content items, e.g., only three to five items. Hence, a huge amount of potential patient experience content items is reduced to a shortlist of favorites that take the target effect into account. The method reduces the list of patient experience content suggestions, such that it is much easier for the patient to select from the list of patient experience content items, while relevant options are ensured to maximize the sense of control. In particular, the selected patient experience content items have the desired effect, i.e., the target effect, on the patients 4. Finally, since the library may be huge, it is unlikely that too little suitable content may be found for specific patient groups, e.g., based on patient properties like age or gender, and/or on properties of the medical procedure, e.g., a scan duration or a type of scan.

Generating S5 the selection of patient experience content items may comprise using a similarity measure of the patient experience content items, such that the selection of patient experience content items comprises types of patient experience content items having little similarity, thus increasing the likelihood that the patient 4 will like at least one of the suggested patient experience content items.

Optionally, the steps of computing low level content properties, deriving S3 high level content effects, and/or prioritizing S4 the patient experience content items may be performed using machine learning, and the machine learning is trained using medical procedure workflow data. In particular, the machine learning may be an artificial intelligence such a neural network. The medical workflow data may comprise data such as a number of rescans needed, a number of scan aborts, or a total scan time. This medical workflow data can then be used as a measure of what content helps the patients 4 to have a successful medical procedure. This is then used as training input for the machine learning system. With said procedure, the real effect on patients 4 during the medical procedure is evaluated and used to determine selections of patient experience content items, which then have an even better positive effect on the patient 4 and/or the medical procedure.

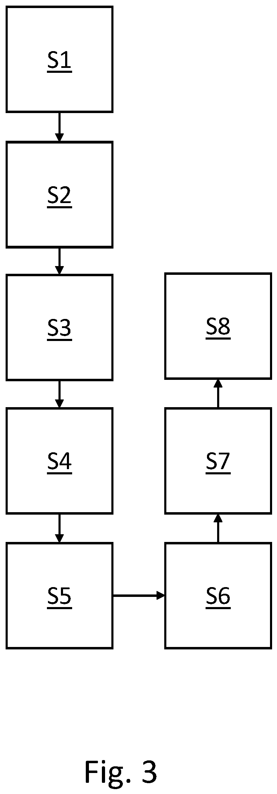

Fig. 3 shows a flowchart of another embodiment of a method for selecting patient experience content. Compared to the method shown in Fig. 2, this method further comprises the steps of presenting S6 the selection of patient experience content items to a patient 4, receiving S7 input on the choice from the patient 4, and providing S8 the chosen patient experience content item to the patient 4 during the medical procedure. Hence, the selection of the patient experience content items is actually used to provide one patient experience content item to the patient 4 during the medical procedure, leading to positive effects on the patient 4 and/or on the result of the medical procedure.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: medical apparatus
- 2: medical device
- 3: patient table
- 4: patient
- 5: patient experience content selection engine
- 6: library
- 7: screen

- S 1: providing a library
- S2: obtaining low level content properties
- S3: deriving high level content effects
- S4: prioritizing the patient experience content items
- S5: generating a selection
- S6: presenting the selection
- S7: receiving input
- S8: providing the patient experience content item

## Claims

1. Method for selecting patient experience content for a medical procedure, comprising:
providing (S 1) a library (6) comprising a plurality of patient experience content items;
obtaining (S2) low level content properties for each of the patient experience content items;
deriving (S3) high level content effects from the low level content properties for each of the patient experience content items;
prioritizing (S4) the patient experience content items based on their high level content effects and a target effect; and
generating (S5) a selection of patient experience content items based on the prioritized (S4) patient experience content items.

2. Method according to claim 1, wherein the medical procedure is a medical imaging procedure, in particular a magnetic resonance imaging, MRI, procedure, a computed tomography, CT, procedure, or an image guided therapy, IGT, imaging procedure, and/or a medical therapy procedure, e.g., an IGT therapy procedure, or a radiotherapy procedure.

3. Method according to claim 1 or 2, wherein the patient experience content comprises visual content, auditory content, olfactory content, and/or tactile content.

4. Method according to any one of claims 1 to 3, wherein the low level content properties comprise color, spatial orientation, velocity, color-change speed, viewpoint orientation, zooms, pans, spatial contrast, color contrast, color tone, flicker, pace, intensity of movement, fades, edge density, average shot length, discontinuous edits, cuts, faces contained, presence of predefined content, language, audio changes, intensity of odor, type of odor, roughness, temperature, hardness, and/or friction.

5. Method according to any one of claims 1 to 4, wherein the step of obtaining (S2) low level content properties comprises computing low level content properties, in particular by an explicit computation engine and/or a trained neural network

6. Method according to any one of claims 1 to 5, wherein the high level content effects comprise physical comfort, calming effects, re-assuring effects, feeling safe effects, sensory indulgence effects, engaging effects, distracting effects, and/or fear-inducing effects.

7. Method according to any one of claims 1 to 6, wherein deriving (S3) the high level content effects is performed using a trained neural network, wherein the neural network has been trained using a plurality of training content items that have been labelled with high level content effects.

8. Method according to any one of claims 1 to 7, wherein deriving (S3) the high level content effects is further based on patient information and/or on situation information.

9. Method according to any one of claims 1 to 8, wherein prioritizing (S4) the patient experience content items is further based on patient preference.

10. Method according to any one of claims 1 to 9, wherein prioritizing (S4) the patient experience content items is further based on medical procedure information, in particular a length of the medical procedure, requirement to lie still, expected patient cooperation, expected noise level, and/or use of contrast.

11. Method according to any one of claims 1 to 10, wherein generating (S5) the selection of patient experience content items comprises using a similarity measure of the patient experience content items, such that the selection of patient experience content items comprises types of patient experience content items having little similarity.

12. Method according to any one of claims 1 to 11, wherein the steps of computing low level content properties, deriving (S3) high level content effects, and/or prioritizing (S4) the patient experience content items are performed using machine learning, and the machine learning is trained using medical procedure workflow data.

13. Method according to any one of claims 1 to 12, further comprising:
presenting (S6) the selection of patient experience content items to a patient (4);
receiving (S7) input on the choice from the patient (4); and
providing (S8) the chosen patient experience content item to the patient (4) during the medical procedure.

14. Patient experience content selection engine (5), configured to perform the method according to any one of claims 1 to 13.

15. Medical apparatus (1) comprising a patient experience content selection engine (5) according to claim 14.
